# FASCICULE DE BREVET EUROPEEN

(11) **EP 1 123 496 B1**
(45) Date de publication et mention de la délivrance du brevet: **01.12.2004**
(21) Numéro de dépôt: 00956640.7
(22) Date de dépôt: 08.08.2000
(51) Int. Cl.: G01N 3/08

(54) **DISPOSITIF POUR LA MESURE DES FORCES DE CONTENTION EXERCEES PAR UNE ORTHESE DE TYPE BAS OU COLLANT DE CONTENTION**
VORRICHTUNG ZUR BESTIMMUNG DER DURCH EINEN KOMPRESSIONSSTRUMPF ODER EINE -STRUMPFHOSE AUSGEÜBTEN KOMPRESSIONSKRÄFTE
DEVICE FOR MEASURING RETENTION FORCES EXERTED BY AN ORTHESIS SUCH AS SUPPORT STOCKINGS OR TIGHTS

(30) Priorité: 10.08.1999 FR 9910366
(43) Date de publication de la demande: 16.08.2001
(73) Titulaire: Innothera Topic International, 94110 Arcueil (FR)
(72) Inventeur: FLAUD, Patrice, F-77380 Combs la Ville (FR); COUNORD, Jean-Louis, F-92500 Rueil Malmaison (FR)
(74) Mandataire: Dupuis-Latour, Dominique
(86) Numéro de dépôt international: PCT/FR2000/002274
(87) Numéro de publication internationale: WO 2001/011337

(56) Documents cités:
- FR-A- 2 764 796
- GB-A- 2 168 156
- US-A- 2 175 661
- US-A- 3 503 257
- US-A- 3 750 291
- US-A- 4 137 763
- US-A- 4 491 255

## Description

L'invention concerne un dispositif pour la mesure extensométrique, non destructive, des forces de contention susceptibles d'être exercées par une orthèse de type bas ou collant de contention (ou, plus exactement, de contention/compression), le terme "bas de contention" étant entendu dans son sens le plus large, incluant les différentes exécutions telles que socquettes, bas jarret ou chaussettes, bas cuisse, bas cuisse autofixants, collants, collants de maternité, collant homme ou hémicollants.

Par "mesure extensométrique", on entendra une mesure consistant à imposer une déformation en un endroit précis du bas et à mesurer la force appliquée localement par le bas sous l'effet de cette déformation, cette force étant fonction des caractéristiques d'élasticité tenant aux matières utilisées et à la structure de l'article.

Il est nécessaire de pouvoir mesurer de façon précise et reproductible la force que permet d'appliquer une orthèse compressive donnée, notamment pour vérifier sa conformité aux valeurs nominales prescrites en fonction de la classe de contention choisie.

Cette mesure est importante notamment au stade de la fabrication, pour vérifier les articles en tombé de métier juste après leur tricotage, pour contrôle de qualité et réglage éventuel du métier à tricoter.

Cette application n'est cependant pas limitative, et l'invention peut être utilisée à de nombreuses autres fins, par exemple pour l'essai de nouvelles matières, le développement de nouveaux produits, les études de fatigue du produit, etc.

Divers protocoles et dispositifs de mesure extensométriques ont déjà été proposés à cette fin par exemple selon la norme allemande RAL-GZ 387, ou comme décrit par le US-A- 4 137 763 (Swallow) ou FR-A-2 764 796 (Innothéra Topic International), qui décrivent une forme reproduisant une jambe et pourvue en surface de capteurs de pression pour une mesure statique, unique, de la compression. Mais ces procédés et dispositifs présentent tous un ou plusieurs des inconvénients suivants :
- destructivité, notamment avec les systèmes utilisant des griffes de traction pour créer une déformation forcée du bas (cf. norme RAL-GZ 387),
- temps de contrôle très long: certains protocoles de mesure nécessitent une durée pouvant aller jusqu'à 48 heures, incompatible avec les , contraintes industrielles de type contrôle de qualité en fabrication,
- nombre réduit de points de mesure alors que, le bas n'étant pas un produit homogène, il est important d'obtenir un nombre aussi élevé que possible de points de mesure sur la totalité de la longueur de l'article,
- coût élevé de l'appareillage,
- nécessité de prévoir, d'une taille de bas à l'autre ou d'un article à l'autre, des gabarits multiples imposant des manipulations importantes.

Le but de la présente invention est de pallier l'ensemble de ces inconvénients, en proposant un dispositif de mesure extensométrique non destructif et adapté (entre autres) au contrôle de qualité en production, par sa robustesse, sa rapidité de mise en oeuvre et sa flexibilité, avec possibilité de s'adapter rapidement et simplement à des articles différents ou des tailles différentes d'un même article.

Le dispositif de l'invention présente en outre les avantages suivants :
- précision de la mesure,
- fidélité de la mesure, afin de fournir de données de manière reproductible et indépendante de l'habilité d'un opérateur,
- mesure simultanée en un grand nombre de points,
- simplicité, rapidité de mise en oeuvre,
- possibilité de numériser, mémoriser, traiter et afficher les différentes données relevées, notamment pour permettre l'interfaçage avec un traitement informatique,
- possibilité de contrôler toutes les tailles standard, toutes les exécutions et toutes les longueurs actuellement produites, ainsi que les articles fabriqués sur mesure.

Le dispositif extensométrique de l'invention est du type général décrit par les US-A-4 137 763 et FR-A-2 764 796 précités, comme visé au préambule de la revendication principale. Il comprend les caractéristiques originales visées par la partie caractérisante de cette revendication. Les sous-revendications énoncent des caractéristiques subsidiaires, avantageuses, de mise en oeuvre de l'invention.

On va maintenant décrire un exemple de mise en oeuvre de l'invention, en référence aux dessins annexés.
La figure 1 est une vue schématique montrant les différents éléments du dispositif de l'invention.
La figure 2 est une vue de détail, en coupe, montrant la structure des capteurs de force.
La figure 3 montre le tracé d'une caractéristique force/allongement.
La figure 4 montre le profil de pression déterminé par le dispositif de l'invention.

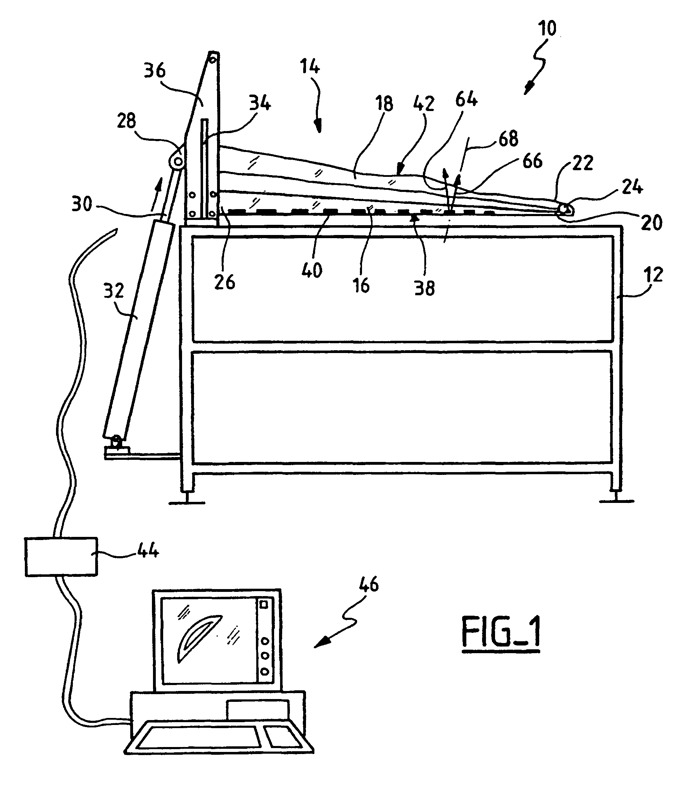

Sur la figure 1, la référence 10 désigne, de façon générale, le dispositif extensométrique de l'invention qui comporte essentiellement, montée sur un bâti 12, une structure déformable 14 destinée à recevoir le bas enfilé sur toute sa longueur (à l'exception du pied).

La structure déformable 14 se compose de deux branches ou "demi-jambes" 16, 18, avec une branche inférieure fixe 16 et une branche supérieure mobile 18, les deux branches étant articulées entre elles à leurs extrémités respectives 20, 22 autour d'un pivot commun 24, à la manière des deux branches d'un compas.

À leurs extrémités opposées 26, 28, les branches 16, 18 sont reliées à des moyens écarteurs permettant de les éloigner l'une de l'autre par un mouvement de rotation relatif autour du pivot 24, cet écartement mutuel étant par exemple réalisé en rendant fixe l'extrémité 26 de la branche inférieure 16 et en déplaçant l'extrémité 28 de la branche supérieure 18 au moyen d'une tige 30 d'un vérin 32, par exemple un vérin électrique programmable en vitesse et en amplitude. Ce vérin électrique est par exemple réalisé à partir d'un moteur pas-à-pas dont la vitesse est ajustable typiquement de 0 à 500 mm/min, et susceptible de procurer une amplitude d'ouverture comprise entre 0 et 400 mm entre les deux bras 16, 18 de la structure déformable 14.

L'extrémité mobile 28 est guidée dans son déplacement par la gorge 34 d'une glissière verticale 36.

La surface externe de la branche inférieure 16 présente un profil rectiligne, et cette branche est pourvue d'une pluralité de capteurs de force 40 répartis sur la longueur de la branche 16 en des points choisis.

Le profil externe 42 de la branche supérieure 18 est un profil curviligne, correspondant à la forme générale d'une jambe.

Pour permettre la mesure d'articles de tailles et de natures différentes, la branche supérieure 18 peut être échangée avec d'autres branches semblables présentant des profils 42 différents. Par ailleurs, le point de rotation 24 peut être adapté, par exemple en rehaussant le point du pivotement pour des tailles de bas avec un diamètre plus important.

Les capteurs 40 sont par exemple au nombre de douze, et sont disposés le long de la branche inférieure 16 de manière à correspondre à la topographie des points de mensuration dite "standard Hohenstein", pour les points b, b1, c, d, e, f, g de ce standard. Avantageusement, les capteurs des points c, d, e, f et g sont dédoublés, ce qui donne un total de douze capteurs pour sept points de mesure.

Selon les articles considérés, une partie seulement de ces capteurs pourra être utilisée, par exemple les capteurs des points b et b1 pour les socquettes, ceux des points b, b1 et c pour les bas jarret, ceux des points b, b1, c, d, e et f pour les bas cuisse, et la totalité des capteurs pour les collants.

Pour que les mesures correspondent bien aux points du bas tels que définis par le standard Hohenstein, il importe de positionner longitudinalement avec précision le bas sur la forme 14, typiquement avec une précision de ± 3 mm, le repère étant pris à partir de la ligne de talon (le dernier tour de trame avant d'arriver au talon), qui sera placée sur un repère approprié gravé sur les branches 16 et 18.

Les différents capteurs 40 déterminent la force appliquée par le bas sur la surface 38, et les valeurs mesurées sont transmises, via une interface 44, à un micro-ordinateur 46 assurant le traitement, le stockage et l'affichage des données recueillies.

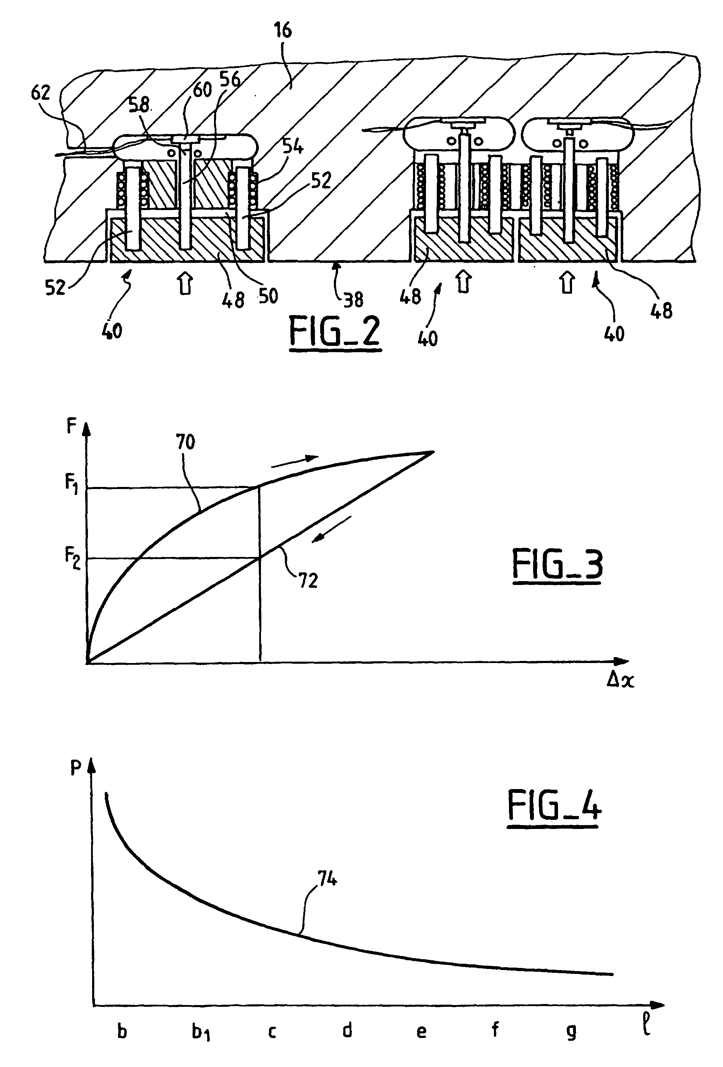

La figure 2 montre le détail de l'un de ces capteurs 40.

Une touche de mesure 48, d'une longueur typique de 30 mm pour un capteur simple (à gauche sur la figure 2) et de 2 x 20 mm pour un capteur double (à droite sur la figure 2), est logée dans une cavité 50 de la branche inférieure 16, la surface de la touche venant affleurer le profil extérieur 38 de cette branche inférieure. Pour réduire au minimum les frottements, la touche 48 est montée sur des guides 52 eux-mêmes associés à

des douilles à billes 54, de manière à n'autoriser un déplacement que dans une direction orthogonale au profil (rectiligne) 38 de la branche 16.

La sollicitation appliquée à la touche 48 est transmise par une tige 56 à un doigt 58 venant en contact avec un capteur de force 60, par exemple un capteur tel que le modèle ELFS-B0-25N-/M1.0,5M/MLL de la marque ENTRAN, qui est un capteur de force à jauge de contrainte présentant une plage de mesure 0-25 N en compression, avec des caractéristiques de non-linéarité, dérive, hystérésis, etc. très faibles. Ce capteur comporte des fils de liaison 62 (alimentation et mesure) cheminant le long du bras inférieur 16 jusqu'à l'interface 44 avec le micro-ordinateur.

Le capteur de force 60 est associé à une chaîne de mesure classique, et donne une indication de la force exercée sur la touche affleurante 48 par le bas enfilé sur l'extensomètre. En rapportant cette force à la surface de la touche, on obtient directement la pression exercée par le bas en ce point.

Le protocole de mesure est le suivant.

Tout d'abord, les deux branches 16, 18 de l'extensomètre étant placées à leur position d'écartement minimal, le bas est enfilé sur l'extensomètre manuellement et librement, c'est-à-dire sans déformation élastique. Le positionnement de la jambe sur l'extensomètre est opéré comme indiqué plus haut à partir de la ligne de talon, le reste du bas étant ensuite déployé, toujours sans déformation élastique, sur la longueur de l'extensomètre (sur tout ou partie de cette longueur, selon qu'il s'agit d'une chaussette, d'un bas, d'un collant, etc.). On notera que, l'extensomètre ne comportant pas de forme de pied à angle droit (contrairement à d'autres extensomètres connus), l'enfilage du bas sur le dispositif est rendu d'autant plus facile et rapide.

Les deux bras sont alors progressivement écartés au moyen de l'actionneur 30, 32, et les signaux recueillis par les capteurs 40 sont tous envoyés en temps réel au système informatique qui enregistre chacune de ces valeurs. Le calcul du déplacement en chaque point de contrôle est effectué par logiciel, en fonction de l'amplitude imposée par l'actionneur.

On notera à ce stade que les valeurs délivrées par chacun des capteurs doivent être traitées pour obtenir, à partir de la force 64 (figure 1) mesurée par le capteur perpendiculairement au profil rectiligne 38 de la branche inférieure 16, la force 66 réellement exercée dans la direction de la ligne de trame 68 du bas, qui est la valeur effective à acquérir. À cet effet, le logiciel d'acquisition des données détermine un facteur correctif approprié pour chaque point de mesure (chaque capteur) et pour chaque pas d'ouverture de la branche mobile 18.

Pour un capteur donné, la force F en fonction de l'allongement transversal Δx du bas présente l'allure illustrée figure 3, avec une portion de caractéristique 70 en phase d'ouverture des branches différente de la portion correspondante 72 en phase de fermeture (effet d'hystérésis). Pour permettre une stabilisation de l'ensemble, le dispositif est commandé de manière à réaliser une succession de cycles d'écartements/rapprochements des deux branches 16, 18, l'acquisition finale des données étant par exemple réalisée au 3^{ème} ou au 6^{ème} cycle. Le nombre de points de mesure pour chaque cycle est typiquement de 500 points pour chacune des deux phases écartement/rapprochement.

On notera que, pour chacun des points de mesure, les données ne sont pas acquises au même moment. En effet, le début de déformation élastique du bas, qui ne commence que lorsque l'allongement imposé par l'extensomètre correspond à la circonférence d'application du bas, est atteint à des ouvertures différentes pour chaque point, et l'ouverture correspondant à la largeur à plat doit être reprise comme référence zéro pour chaque point.

Du fait de l'hystérésis, une même valeur d'allongement pourra donner deux valeurs de force F1 et F2 différentes ; on prendra la moyenne comme valeur de mesure.

Le logiciel pourra, à partir des différentes données relevées, évaluer un certain nombre de paramètres tels que :
- force de contention P en chaque point,
- rigidité en chaque point,
- dégressivité en chaque point par rapport à la cheville,
- écart de dégressivité entre différents points.

Ces données pourront être affichées, par exemple sur écran, en même temps que des paramètres tels que numéro de lot, numéro d'essai, données statistiques (moyenne et écart-type au sein d'un lot), etc.

La visualisation des caractéristiques force/allongement (figure 3) pour chaque point de mesure permet de détecter visuellement des anomalies et de rejeter les mesures douteuses.

Le dispositif pourra également afficher sur écran le profil des pressions du bas testé (figure 4), donnant en fonction des différents points de mesure b, b1, c... répartis sur la longueur f du bas, les valeurs correspondantes des pressions P exercées par celui-ci.

## Revendications

1. Un dispositif (10) pour la mesure extensométrique, non destructive, des forces de contention susceptibles d'être exercées par une orthèse de type bas ou collant de contention, comportant:
- une forme (14) apte à recevoir une orthèse enfilée dessus,
- des capteurs (40), propres à mesurer la force appliquée localement par l'orthèse à l'endroit du capteur respectif,
dispositif **caractérisé en ce que** ladite forme comprend deux branches allongées (16,18) et les capteurs sont répartis sur la longueur de l'une au moins des branches pour mesurer la force appliquée localement par l'orthèse selon une composante perpendiculaire au profil de la branche,
et **en ce qu'**il comporte en outre:
- des moyens écarteurs (30, 32), coopérant avec des extrémités en vis-à-vis (26, 28) des branches pour éloigner celles-ci transversalement à leur plus grande dimension, et
- des moyens de commande des moyens écarteurs, propres à éloigner les branches dans un mouvement d'écartement mutuel, progressif et contrôlé.

2. Le dispositif de la revendication 1, dans lequel les branches sont réunies à pivotement à leurs extrémités (20, 22) opposées aux extrémités coopérant avec les moyens écarteurs, le mouvement d'écartement mutuel étant un mouvement autour du point de pivotement correspondant.

3. Le dispositif de la revendication 1, dans lequel les capteurs (40) comportent comme élément sensible en contact avec l'orthèse des touches (48) affleurant le profil externe (38) de la branche sur laquelle ils sont placés.

4. Le dispositif de la revendication 1, dans lequel les moyens écarteurs comprennent une branche rectiligne (16) portant les capteurs et une branche curviligne (18) dont le profil correspond à celui d'une jambe destinée à recevoir l'orthèse.

5. Le dispositif de la revendication 1, dans lequel les moyens écarteurs (30, 32) sont des moyens motorisés.

6. Le dispositif de la revendication 1, comportant des moyens pour déterminer l'allongement transversal (Δx) de l'orthèse à l'endroit de chacun des capteurs en fonction de l'écartement des branches appliqué par les moyens écarteurs.

7. Le dispositif de la revendication 5, comprenant des moyens pour enregistrer, pour chaque capteur, une pluralité de couples de mesure force (F)/allongement (Δx).

8. Le dispositif des revendications 4 et 6 prises en combinaison, comprenant des moyens pour déterminer dynamiquement une caractéristique force/allongement.

9. Le dispositif de la revendication 1, comprenant des moyens pour déterminer le profil des pressions de contention (P) exercées par l'orthèse sur la longueur (ℓ) de celle-ci.

## Patentansprüche

1. Vorrichtung (10) zur zerstörungsfreien, extensometrischen Messung der Kompressionskräfte, die imstande sind, durch eine Orthese vom Typ Strumpf oder Kompressionsstrumpfhose ausgeübt zu werden, umfassend:
- eine Form (14), geeignet, eine Orthese zu empfangen, die auf diese aufgezogen wird,
- Messfühler (40), geeignet, die Kraft zu messen, die lokal durch die Orthese am Ort des jeweiligen Messfühlers angewandt wird,
wobei die Vorrichtung **dadurch gekennzeichnet ist, dass** die Form zwei längliche Arme (16, 18) umfasst und die Messfühler auf der Länge mindestens eines der Arme verteilt sind, um die Kraft zu messen, die lokal durch die Orthese ausgeübt wird entlang einer Komponente, die senkrecht zum Profil des Arms liegt,
und dass sie ferner umfasst:
- Spreizmittel (30, 32), die mit den gegenüberliegenden Enden (26, 28) der Arme zusammenwirken, um diese transversal bis zu ihrem größten Ausmaß voneinander zu entfernen, und
- Mittel zu Steuerung der Spreizmittel, geeignet, die Arme in einer Bewegung der gegenseitigen, allmählichen und kontrollierten Spreizung voneinander zu entfernen.

2. Vorrichtung gemäß Anspruch 1, in welcher die Arme drehend an ihren Enden (20, 22) miteinander verbunden sind, die den Enden gegenüberliegen, welche mit den Spreizmitteln zusammenwirken, wobei die Bewegung der gegenseitigen Entfernung voneinander eine Bewegung um den entsprechenden Drehpunkt ist.

3. Vorrichtung gemäß Anspruch 1, in welcher die Messfühler (40) als empfindliches Element in Kontakt mit der Orthese Tasten (48) umfassen, die mit dem externen Profil (38) des Arms fluchten, auf welchem sie platziert sind.

4. Vorrichtung gemäß Anspruch 1, in welcher die Spreizmittel einen gradlinigen Arm (16) umfassen, welcher die Messfühler trägt und einen gekrümmten Arm (18), dessen Profil demjenigen eines Beins entspricht, welches die Orthese empfangen soll.

5. Vorrichtung gemäß Anspruch 1, in welcher die Spreizmittel (30, 32) motorgetriebene Mittel sind.

6. Vorrichtung gemäß Anspruch 1, umfassend Mittel zur Bestimmung der transversalen Verlängerung (Δx) der Orthese am Ort jedes der Messfühler, abhängig von der durch die Spreizmittel angewandten Spreizung der Arme.

7. Vorrichtung gemäß Anspruch 5, umfassend Mittel zum Aufzeichnen, für jeden Messfühler, einer Vielzahl von Messmomenten Kraft (F)/Verlängerung (Δx).

8. Vorrichtung gemäß Ansprüchen 4 und 6, in Kombination genommen, umfassend Mittel zur dynamischen Bestimmung einer Kraft/Verlängerungs-Charakteristik.

9. Vorrichtung gemäß Anspruch 1, umfassend Mittel zum Bestimmen des Profils der Kompressionsdrücke (P), die durch die Orthese über die Länge (1) derselben ausgeübt werden.

## Claims

1. An apparatus (10) for non-destructive extensometric measurement of the support forces that can be exerted by an orthesis of the support hose or tights type, comprising:
- a jig (14) suitable for receiving an orthesis engaged thereon,
- sensors (40) suitable for measuring the force applied locally by the orthesis at the location of the respective sensor,
the apparatus being **characterized in that** said jig includes two elongate branches (16, 18) and the sensors are distributed along the length of at least one of the branches in order to measure the force applied locally by the orthesis in terms of a component perpendicular to the profile of the branch,
and **in that** it further includes :
- expander means (30, 32) co-operating with facing ends (26, 28) of the branches to move them apart from each other transversely to their long dimension, and
- means for controlling the expander means, suitable for moving the branches so that they move apart from each other progressively and in controlled manner.

2. The apparatus of claim 1, in which the branches are pivotally united at their ends (20, 22) remote from their ends which co-operate with the expander means, the movement of mutually moving apart from each other being a pivoting movement about the corresponding pivot point.

3. The apparatus of claim 1, in which the sensors (40) include as sensor elements in contact with the orthesis buttons (48) that lie flush with the outside profile (48) of the branch in which they are located.

4. The apparatus of claim 1, in which the expander means comprise a rectilinear branch (16) carrying the sensors and a curvilinear branch (18) whose profile corresponds to that of a leg suitable for receiving the orthesis.

5. The apparatus of claim 1, in which the expander means (30, 32) are motorized means.

6. The apparatus of claim 1, including means for determining the transverse elongation (□x) of the orthesis at the location of each of the sensors as a function of the spacing imposed on the branches by the expander means.

7. The apparatus of claim 5, including means for recording a plurality of pairs of measurements for each sensor, each pair comprising a force measurement (F) and an elongation measurement (□x).

8. The apparatus of claims 4 and 6 in combination, including means for dynamically determining a force/elongation characteristic.

9. The apparatus of claim 1, including means for determining the support pressure profile (P) exerted by the orthesis over the length (ℓ) thereof.
